# EUROPEAN PATENT APPLICATION

(11) **EP 1 222 918 A1**
(43) Date of publication of application: **17.07.2002**
(21) Application number: 00971451.0
(22) Date of filing: 19.10.2000
(51) Int. Cl.: A61K 9/50, A61K 7/00, A23P 1/04

(54) **MICROCAPSULES FOR STABILIZING COSMETIC, PHARMACEUTICAL OR FOOD PRODUCTS**

(30) Priority: 21.10.1999 ES 9902323
(71) Applicant: Lipotec, S.A., 08902 Hospitalet de Llobregat (Barcelona) (ES)
(72) Inventor: PARENTE DUENA, Antonio, E-08960 San Just Desvern (ES); BONILLA MUNOZ, Angel, E-8830 Sant Boi de Llobregat (ES); GARCES GARCES, Josep, E-8760 Martorell (ES)
(74) Representative: Davila Baz, Angel
(86) International application number: ES0000403
(87) International publication number: WO0128530

(57) **Abstract**

Microcapsules for stabilisation of cosmetic, pharmaceutical or food products, of a size smaller than 500 µm, comprising a nucleus of an adsorbent material -natural or modified polysaccharide insoluble in water, or an adsorbent inorganic material- in which are contained the active ingredients and is coated by a polymeric material -natural , modified natural or synthetic polymer- apt for use in the cosmetics, pharmaceutics or food fields.

Use of said microcapsules, characterised by their incorporation in cosmetic products such as gels, creams, lotions, emulsions, bath gels, shampoos and the like; incorporation in pharmaceutical and veterinary products applied topically, orally or parenterally; incorporation in food products for human or animal consumption and in diet products.

## Description

The present invention relates to a new type of microcapsules for stabilisation of active principles, of application in the field of cosmetics, pharmaceutics or food products, as well as to its method of preparation.

According to the context of the invention, the term microcapsules may encompass the following terms: microparticles, millispheres, microspheres. However, for the purposes of this invention and in order to facilitate its comprehension the term microcapsules shall be employed.

A point of growing interest in the world of cosmetics, pharmaceutics and food is the use in cosmetics, pharmaceutics and food or diet products of substances with several nutritional, pharmaceutical properties o which can cause physiological reactions (such as cholesterol reducers, relaxants, stimulants, antioxidants, vitamin supply, enzyme supply, etc.). However, use of these substances dissolved freely within the product volume may bring about strange flavours or odours which are hard to mask, or the instability and degradation of the substance.

The inclusion or not of these substances within microcapsules can help to deal with these disadvantages. Said solution has been widely employed in the pharmaceutical and cosmetic fields, using millicapsules of amylose (WO 89/11269), millicapsules of glycolic acid (EP 0202159), microcapsules of gelatine and alginic acid (WO 87/01587), microcapsules of ateloglycanes (WO 92/02254), millicapsules of calcium alginate (EP 0391803).

Research has discovered that products of interest in the cosmetics, pharmaceutics and food fields can be advantageously encapsulated inside microcapsules consisting of a nucleus of an adsorbent material and a coating of polymeric material. Products encapsulated in this type of microcapsules show a high stability to degradation agents, regardless of whether the environment of the microcapsules is aqueous, oily or emulsion.

The object of the present invention is thus a cosmetic, pharmaceutical and food preparation to incorporate active principles of interest to cosmetics, pharmaceutics and food products, characterised in that the product of interest is encapsulated within microcapsules comprising a nucleus of an adsorbent material and a polymeric material coating.

The present invention comprises the preparation and use of microcapsules of size smaller than 500 µm, formed of an adsorbent material nucleus in which is incorporated the active substance or product of interest, covered by a polymeric material membrane.

Incorporation of the active product in the nucleus of microcapsules is performed in the following manner:
a) Solution of the product to be encapsulated in a suitable solvent (water, ethanol,...);
b) Incorporation of the product to be encapsulated in the adsorbent material by wetting the adsorbent material with the solution obtained from the previous step;
c) Drying the adsorbent material nucleus obtained in the previous step by conventional processes in order to eliminate the solvent; and
d) Coating the nucleus of adsorbent material incorporating the encapsulated product with a solution of polymeric material.

By adsorbent material is meant all materials of natural or synthetic origin insoluble in water which may incorporate product inside the pores of its particles. More specifically, the following products are seen as adsorbent materials:
- Natural or modified polysaccharides insoluble in water (specifically starch, agarose, cellulose, modified celluloses and their mixtures).
- Inorganic adsorbent materials (more specifically talcum, silica, diatomite earth, active carbon and their mixtures).

Polymeric coating materials are such as the following materials:
- Natural or modified natural polymers which may form films by evaporation of their solutions, such as ethylcellulose, hydroxypropylmethyl cellulose, cellulose acetylphtalate, cellulose hydroxypropylmethylphtalate and the like;
- Synthetic polymers suitable for use in cosmetics, pharmaceutics or foods which may form films by evaporation of their solutions, such as methacrylic acid polymers (Eudragit L and S), copolymers of dimethylaminoethylmetacrylate (Eudragit E), copolymers of trimethylamonioethylmetacrylate (Eudragit RL and RS) and their mixtures, polymers and copolymers of lactic and glycolic acids and their mixtures.

Compounds of interest in the fields of cosmetics, pharmaceutics and food products which may be incorporated to the preparation object of this invention include but are not limited to the following:
- Bactericide drugs such as gentamicine;
- Antiviral agents such as riphampacine or acyclovir;
- Fungicidal agents such as anphotericine B, myconazol, terconazol, econazol, isoconazol, thioconazol, biphonazol, chlotrimazol, ketoconazol, butaconazol, itraconazol, oxyconazol, phenticonazol, nystatin, naphtifine, zinoconazol, cyclopyroxolamine;
- Antiparasite compounds such as those derived from antimony;
- Antitumoral and antineoplasic compounds such as adriamycine, vinblastine, vincristine, mitomycine C, doxorubicine, daunorubicine, methotrexate, cysplatinum and others;
- Antimetabolytes;
- Proteins such as albumin;
- Toxins such as diphtheric toxin;
- Enzymes such as catalase;
- Peptides such as cyclosporine A, hirudine, somatostatin or thymopentin;
- Hormones such as oestrogen;
- Peptidic hormones such as human growth hormone, porcine growth hormone, bovine growth hormone, human calcitonin, salmon calcitonin, carbocalcitonin and insulin;
- Hormone antagonists;
- Neurotransmitters such as acetylcholine;
- Neurotransmitter antagonists;
- Glycoproteins such as hyaluronic acid;
- Lipoproteins such as alpha-lipoprotein;
- Immunoglobulins such as IgG;
- Immunomodulators such as interferon or interleukin;
- Vasodilators;
- Colorants such as Arsenaze III;
- Radioactive markers such as ¹⁴C;
- Radio opaque compounds such as ⁹⁰Te;
- Fluorescent compounds such as carboxyfluorescine;
- Cell receptors such as the oestrogen receptor protein;
- Non-steroid anti-inflammatories such as indomethacine, ibuprofen, sulindac, pyroxicam and naproxen;
- Anti-inflammatories such as dexametasone;
- Anti glaucoma agents such as pilocarpin or thymolol;
- Mydriatic compounds;
- Local anaesthetics such as lidocaine;
- Narcotics such as codeine;
- Vitamins such as alpha-tocopherol;
- Nucleic acids such as thiamine;
- Polynucleotides such as RNA;
- Psychoactive or ansiolitic compounds such as diazepam;
- Mono-, di- and polysaccharides such as glycogen;
- Glycosaminoglycanes such as non fractioned heparins, low molecular mass heparins, pentasaccharide, dermatan sulphate, heparan sulphate, chondroitin-4-sulphate, chondroitin-6-sulphate and their derivatives;
- Cardiovascular agents such as alpha blockers, beta blockers, calcium, channel blockers, ACE inhibitors;
- Prostaglandins;
- Vegetable oils such as vine oil, borage oil, castor oil, olive oil, sunflower oil, and the like;
- Animal oils such as cod liver oil, fish oils, EPA-18, DHA-22 and the like;
- Cosmetic use silicones;
- Vegetable extracts such as those of ging-seng, arnica, calendula, and the like;
- Enzymes and co-enzymes such as ubidecarenone and the like;
- Animal extracts;
- Yeasts such as selenium yeast, beer yeast and the like;
- Vitamins and their derivatives such as vitamin E, vitamin E acetate, vitamin A palmitate, and the like;
- Amino acids such as cystine and the like;
- Animal proteins such as ovoalbumin, gelatine and the like,
- Vegetable proteins such as gluten and the like;
- Hydrolysed animal or vegetable proteins such as hydrolysed collagen or hydrolysed wheat gluten and the like;
- Natural polysaccharides such as hyaluronic acid and the like;
- Natural tocopherols and their mixtures such as Lipatra 2050 and the like;
- Stimulants such as caffeine and the like;
- Mineral and trace element salts, such as iron salts, zinc salts, selenium salts and the like;
- Colorants accepted in the field of cosmetics, dermopharmaceutics or food.

Microcapsules of the above described preparation may be used in:
- cosmetic products such as gels, creams, lotions, emulsions, bath gels, shampoos;
- pharmaceutical and veterinary products by topical, oral or parenteral intake;
- products meant for human or animal consumption and diet products.

Below are presented several examples of preferred embodiments of the present invention:

### Example 1: Obtaining microcapsules containing docosahexenoic acid (DHA):

In a suitable container are dissolved 5 g of DHA in 10 mL CH₂Cl₂; the resulting solution is added shaken onto 95 g of corn starch. Later the solvents are eliminated to obtain a starch powder containing DHA.

3 g of ethylcellulose are dissolved in 25 mL of ethanol, and the resulting solution is added in five different fractions to starch powder containing DHA previously prepared, as follows:
1) Addition of aliquot of ethylcellulose solution on the starch powder;
2) Shaking for ten minutes to obtain a homogenous system;
3) Drying the resulting product;
4) Repetition of step I until the entire ethylcellulose solution is used.

| **Example no.** | **Active material** | **Adsorbent material** | **Coating material** |
|---|---|---|---|
| 2 | Retinol (Vitamin A) | Corn starch | Ethylcellulose |
| 3 | Ascorbic acid | Talcum | Ethylcellulose |
| 4 | Beta-carotene | Microcrystalline cellulose | Eudragit S |
| 5 | Pancreatin | Talcum | Cellulose acetophtalate |
| 6 | Ubidecarenone | Diatomite earth | Lactic-co-glycolic polymer, molecular mass 50000 and lactic glycolic ratio 1:1 |
| 7 | Lipase | Corn starch | Ethylcellulose |
| 8 | Salicylic acid | Corn starch | Ethylcellulose |
| 9 | Hydrolysed wheat | Talcum | Cellulose |
| | protein (Pronalen Flash tense) | | acetophtalate |
| 10 | Hydroglycolic extract of fruits (Pronalen Bio protect) | Corn starch | Ethylcellulose |
| 11 | Piperite mint essence | Microcrystalline cellulose | Ethylcellulose |
| 12 | Perfume | Corn starch | Ethylcellulose |

## Claims

1. Microcapsules for stabilisation of cosmetic, pharmaceutical or food products, **characterised in that** they comprise a nucleus of an adsorbent material, in which are included the active ingredients, and coated in a polymeric material.

2. Microcapsules for stabilisation of cosmetic, pharmaceutical or food products, as claimed in claim 1, **characterised in that** their size is not smaller than 500 µm.

3. Microcapsules for stabilisation of cosmetic, pharmaceutical or food products, as claimed in claims 1 and 2, **characterised in that** the adsorbent material is a natural or modified polysaccharide insoluble in water.

4. Microcapsules for stabilisation of cosmetic, pharmaceutical or food products, as claimed in claims 1 to 3, **characterised in that** the adsorbent material is starch, agarose, cellulose, modified cellulose and their mixtures.

5. Microcapsules for stabilisation of cosmetic, pharmaceutical or food products, as claimed in claims 1 and 2, **characterised in that** the adsorbent material is an adsorbent inorganic material.

6. Microcapsules for stabilisation of cosmetic, pharmaceutical or food products, as claimed in claims 1 and 5, **characterised in that** the adsorbent material is talcum, silica, diatomite earth, active carbon and their mixtures.

7. Microcapsules for stabilisation of cosmetic, pharmaceutical or food products, as claimed in claims 1 to 6, **characterised in that** the polymeric coating material is a natural polymer or a modified natural polymer which may be used in the cosmetic, pharmaceutical or food field, which may form films.

8. Microcapsules for stabilisation of cosmetic, pharmaceutical or food products, as claimed in claims I to 7, **characterised in that** the polymeric coating material is ethylcellulose, hydroxypropylmethylcellulose, cellulose acetophtalate and cellulose hydrohypropylmethyl phtalate.

9. Microcapsules for stabilisation of cosmetic, pharmaceutical or food products, as claimed in claims 1 to 6, **characterised in that** the polymeric coating material used is a synthetic polymer which may be used in the cosmetic, pharmaceutical or food field, which may form films by evaporation of its solutions.

10. Microcapsules for stabilisation of cosmetic, pharmaceutical or food products, as claimed in claims 1 to 6 and 9, **characterised in that** the polymeric coating material are polymers of methylacrylic acid (Eudragit L and S), copolymers of dimethylaminoethylmetacrylate (Eudragit E) and copolymers of dimethylamonioethylmetacrylate (Eudragit RL and RS) and their mixtures, copolymers of glycolic and lactic acids and their mixtures.

11. Microcapsules for stabilisation of cosmetic, pharmaceutical or food products, as claimed in claims 1 to 10, **characterised in that** the active ingredients of cosmetic, pharmaceutical and food interest are:
- Bactericide drugs such as gentamicine;
- Antiviral agents such as riphampacine or acyclovir;
- Fungicidal agents such as anphotericine B, myconazol, terconazol, econazol, isoconazol, thioconazol, biphonazol, chlotrimazol, ketoconazol, butaconazol, itraconazol, oxyconazol, phenticonazol, nystatin, naphtifine, zinoconazol, cyclopyroxolamine;
- Antiparasite compounds such as those derived from antimony;
- Antitumoral and antineoplasic compounds such as adriamycine, vinblastine, vincristine, mitomycine C, doxorubicine, daunorubicine, methotrexate, cysplatinum and others;
- Antimetabolytes;
- Proteins such as albumin;
- Toxins such as diphtheric toxin;
- Enzymes such as catalase;
- Peptides such as cyclosporine A, hirudine, somatostatin or thymopentin,
- Hormones such as oestrogen;
- Peptidic hormones such as human growth hormone, porcine growth hormone, bovine growth hormone, human calcitonin, salmon calcitonin, carbocalcitonin and insulin;
- Hormone antagonists;
- Neurotransmitters such as acetylcholine;
- Neurotransmitter antagonists;
- Glycoproteins such as hyaluronic acid;
- Lipoproteins such as alpha-lipoprotein;
- Immunoglobulins such as IgG;
- Immunomodulators such as interferon or interleukin;
- Vasodilators;
- Colorants such as Arsenaze III;
- Radioactive markers such as ¹⁴C;
- Radio opaque compounds such as ⁹⁰Te;
- Fluorescent compounds such as carboxyfluorescein;
- Cell receptors such as the oestrogen receptor protein;
- Non-steroid anti-inflammatories such as indomethacine, ibuprofen, sulindac, pyroxicam and naproxen;
- Anti-inflammatories such as dexametasone;
- Anti glaucoma agents such as pilocarpine or thymolol;
- Mydriatic compounds;
- Local anaesthetics such as lidocaine;
- Narcotics such as codeine;
- Vitamins such as alpha-tocopherol;
- Nucleic acids such as thiamine;
- Polynucleotides such as RNA;
- Psychoactive or ansiolitic compounds such as diazepam;
- Mono-, di- and polysaccharides such as glycogen;
- Glycosaminoglycanes such as non fractioned heparins, low molecular mass heparins, pentasaccharide, dermatan sulphate, heparan sulphate, chondroitin-4-sulphate, chondroitin-6-sulphate and their derivatives;
- Cardiovascular agents such as alpha blockers, beta blockers, calcium, channel blockers, ACE inhibitors;
- Prostaglandins;
- Vegetable oils such as vine oil, borage oil, castor oil, olive oil, sunflower oil, and the like;
- Animal oils such as cod liver oil, fish oils, EPA-18, DHA-22 and the like;
- Cosmetic use silicones;
- Vegetable extracts such as those of ging-seng, arnica, calendula, and the like;
- Enzymes and co-enzymes such as ubidecarenone and the like;
- Animal extracts;
- Yeasts such as selenium yeast, beer yeast and the like;
- Vitamins and their derivatives such as vitamin E, vitamin E acetate, vitamin A palmitate, and the like;
- Amino acids such as cystine and the like;
- Animal proteins such as ovoalbumin, gelatine and the like,
- Vegetable proteins such as gluten and the like;
- Hydrolysed animal or vegetable proteins such as hydrolysed collagen or hydrolysed wheat gluten and the like;
- Natural polysaccharides such as hyaluronic acid and the like;
- Natural tocopherols and their mixtures such as Lipatra 2050 and the like;
- Stimulants such as caffeine and the like;
- Mineral and trace element salts, such as iron salts, zinc salts, selenium salts and the like;
- Colorants accepted in the fields of cosmetics, dermopharmaceutics, or food.

12. Use of microcapsules as claimed in claims I to 11, **characterised by** their incorporation into cosmetic products such as gels, creams, lotions, emulsions, bath gels, shampoos and the like.

13. Use of microcapsules as claimed in claims 1 to 11, **characterised by** their incorporation into pharmaceutical and veterinary products applied topically, orally or parenterally.

14. Use of microcapsules as claimed in claims 1 to 11, **characterised by** their incorporation into food products for human or animal consumption and in diet products.
